# EUROPÄISCHE PATENTANMELDUNG

(11) **EP 0 714 880 A1**
(43) Veröffentlichungstag der Anmeldung: **05.06.1996**
(21) Anmeldenummer: 95118047.0
(22) Anmeldetag: 16.11.1995
(51) Int. Cl.: C07C 49/683, C07C 49/747, C07C 49/753, C07D 453/02, C07D 235/18, C07C 309/24, A61K 31/12, A61K 7/42

(54) **Bis(methyliden)-phenylen-Derivate**

(30) Priorität: 29.11.1994 DE 4442324
(71) Anmelder: MERCK PATENT GmbH, D-64293 Darmstadt (DE)
(72) Erfinder: Stein, Inge, Dr., D-63110 Rodgau (DE); Schwarz, Michael, Dr., D-64521 Gross-Gerau (DE); Heywang, Ulrich, Dr., D-64289 Darmstadt (DE); Kompter, Michael, Dr., D-64560 Riedstadt (DE)

(57) **Zusammenfassung**

Die Erfindung betrifft Bismethylidenphenylen-Derivate der Formel I,

A¹-Phe-A² I

worin
- Phe: eine unsubstituierte oder durch 1 bis 4 Hydroxy-, Alkyl- oder Alkoxygruppen mit 1 bis 10 C-Atomen substituierte Phenylengruppe,
- A¹: eine Gruppe ausgewählt aus der Formel 1, 2, 3 und 4

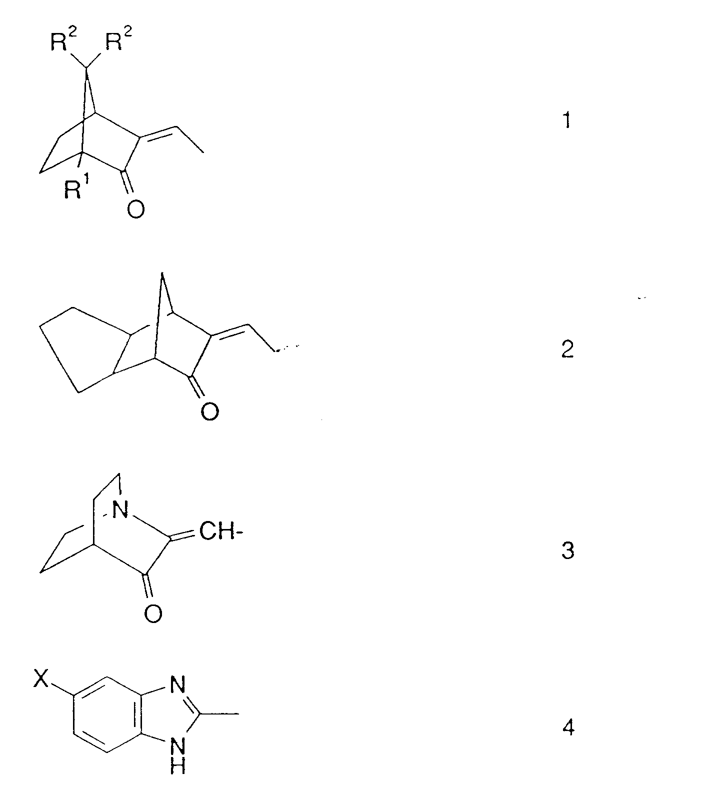

worin
- R¹: H, CH₃ oder -CH₂SO₃H
- R²: H oder CH₃ und X H oder SO₃H bedeuten
und
- A²: eine von A¹ verschiedene Gruppe ausgewählt aus den Formeln 1, 2, 3 und 4
bedeuten,
sowie deren Herstellung und Verwendung als UV-Filter insbesondere in kosmetischen oder pharmazeutischen Zubereitungen.

## Beschreibung

Die Erfindung betrifft Bismethylidenphenylen-Derivate der Formel I,

A¹-Phe-A² I

worin
- Phe: eine unsubstituierte oder durch 1 bis 4 Hydroxy-, Alkyl- oder Alkoxygruppen mit 1 bis 10 C-Atomen substituierte Phenylengruppe,
- A¹: eine Gruppe ausgewählt aus den Formeln 1, 2, 3 und 4
bedeuten, 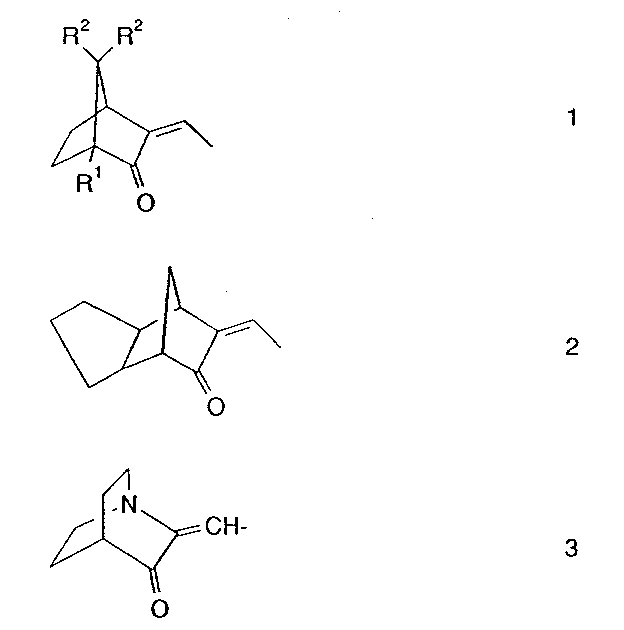 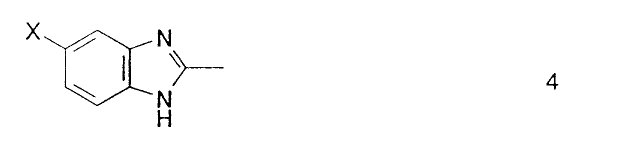
worin
- R¹: H, CH₃ oder -CH₂SO₃H
- R²: H oder CH₃, und X H oder SO₃H bedeuten,
und
- A²: eine von A¹ verschiedene Gruppe ausgewählt aus den Formeln 1, 2, 3 und 4,
bedeuten,
sowie Verfahren zu ihrer Herstellung und ihre Verwendung in kosmetischen Zubereitungen, insbesondere zum Schutz vor Sonnenstrahlung, und in pharmazeutischen Zubereitungen zur vorbeugenden Behandlung von Entzündungen und Allergien der Haut oder bestimmter Krebsarten.

Bekanntlich reagiert die Haut empfindlich auf Sonnenstahlen, welche einen gewöhnlichen Sonnenbrand oder ein Erythem, aber auch mehr oder weniger ausgeprägte Verbrennungen hervorrufen können.

Sonnenstrahlen haben aber auch andere negative Wirkungen: sie bewirken, daß die Haut ihre Elastizität verliert und sich Falten bilden und führen somit zu einer frühzeitigen Alterung. Manchmal kann man auch Dermatosen beobachten. Im estremen Fall kommt es bei manchen Menschen zum Auftreten von Hautkrebs.

Es ist auch wünschenswert, Haare gegen photochemische Schäden zu schützen, um Veränderungen von Farbnuancen, ein Entfärben oder Schäden mechanischer Art zu verhindern.

Es ist bekannt, daß die in kosmetischen Präparaten enthaltenen Komponenten nicht immer genügend lichtstabil sind und sich unter der Einwirkung von Lichtstrahlen zersetzen.

Bekanntlich wird der gefährlichste Teil der Sonnenstrahlen von den ultravioletten Strahlen mit einer Wellenlänge von weniger als 400 nm gebildet. Bekannt ist auch, daß durch das Vorhandensein der Ozonschicht der Erdatmosphäre, die einen Teil der Sonnenstrahlung absorbiert, die untere Grenze der ultravioletten Strahlen, welche die Erdoberfläche erreichen, bei ca. 280 nm liegt.

Es erscheint somit wünschenswert, Verbindungen zur Verfügung zu stellen, welche UV-Strahlen in einem Wellenlängenbereich von 280 bis 400 nm absorbierten können, d.h. auch UV-B-Strahlen mit einer Wellenlänge zwischen 280 und 320 nm, die bei der Bildung eines Sonnenerythems eine entscheidende Rolle spielen, wie auch UV-A-Strahlen mit einer Wellenlänge zwischen 320 und 400 nm, welche die Haut bräunen aber auch altern lassen, die Auslösung einer erythematösen Reaktion begünstigen oder diese Reaktion bei bestimmten Menschen vergrößern oder sogar phototoxische oder photoallergische Reaktionen auslösen können.

Die heute üblichen Sonnenschutzfilter in der Kosmetik werden in UVA- bzw. UVB-Filter unterteilt. Während es im UVB-Bereich (280-320 nm) mit Substanzen wie Eusolex® 6300 oder Eusolex® 232 gute Filter gibt, sind die im UVA-Bereich (320-400 nm) verwendeten problembehaftet:

Dibenzoylmethane wie Parsol® 1789 oder Eusolex® 8020 sind bei UV-Bestrahlung nicht unbegrenzt stabil, was zum einen die Filtereffektivität mit der Zeit herabsetzt und zum anderen Photosensibilisierungen der Haut in vereinzelten Fällen begünstigen kann. Die ebenfalls als UVA-Filter verwendeten Benzophenone sind in den in der Kosmetik verwandten Ölen nur begrenzt löslich, und sie weisen eine relativ geringe Absorption auf.

Dagegen sind nur wenige wasserlösliche UVA-Filter derzeit bekannt, deren UV-Absorption jedoch gering ist

Ähnliche Benzylidencampher-Derivate sind z.B. aus der EP 0 390 682 bekannt; diese weisen jedoch keine Tricyclodecanon bzw. Chinuclidinon-Gruppe auf.

In der WO 93/16978 werden ähnliche Verbindungen mit nur einer Ketotricyclo[5.2.1.0]decan-Gruppe (2) beschrieben.

In der EP 0 576 974 werden ähnliche Verbindungen mit nur einer Chinuclidinon-Gruppe (3) beschrieben.

Diese weisen jedoch relativ niedrige Löslichkeiten in organischen Lösungsmitteln auf.

Es wurde gefunden, daß Derivate der Formel I, worin Phe eine unsubstituierte oder durch 1 bis 4 Hydroxy-, Alkyl- oder Alkoxygruppen substituierte Phenylengruppen bedeutet, insbesondere eine unsubstituierte 1,4-Phenylengruppe bedeutet, hervorragende UVA-Filter-Eigenschaften besitzen. Ihre Löslichkeit in den in der Kosmetik verwandten Ölen ist sehr gut, so daß Einsatzkonzentrationen auch in komplizierten Formulierungen bis mindestens 10 % der Zubereitung möglich sind.

Weiterhin weisen die erfindungsgemäßen Verbindungen eine außerordentliche Photostabilität gegenüber UV-Strahlung auf, die die Stabilität bisher bekannter UV-Filtersubstanzen bei weitem übersteigt, sie eignen sich insbesondere als UVA- oder UV-Breitbandfilter.

Falls die Extinktion im UVB-Bereich ein Minimum aufweist, ist das kein Nachteil, da man problemlos einen UVB-Filter mit in die Formulierung einarbeiten kann.

Weiterhin können die Verbindungen der Formel I auch zur vorbeugenden Behandlung von Entzündungen und Allergien der Haut und zur Verhütung bestimmter Krebsarten verwendet werden.

Außer ihren guten Eigenschaften als Filter zeichnen sich die erfindungsgemäßen Verbindungen durch eine gute thermische und photochemische Stabilität aus.

Ferner bieten diese Verbindungen den Vorteil, nicht toxisch oder reizend und gegenüber der Haut vollkommen unschädlich zu sein.

Sie verteilen sich gleichmäßig in den herkömmlichen kosmetischen Trägern und können insbesondere in Fett-Trägern einen kontinuierlichen Film bilden; sie können auf diese Weise auf die Haut aufgetragen werden, um einen wirksamen Schutzfilm zu bilden.

Gegenstand der Erfindung sind die Verbindungen der oben gegebenen Formel I, insbesondere worin Phe unsubstituiertes oder durch 1 bis 2 Alkoxygruppen mit 1 bis 2 C-Atomen substituiertes 1,4-Phenylen bedeutet.

Vorzugsweise ist Phe eine Gruppe der Formel 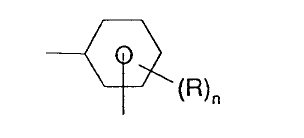
insbesondere eine Gruppe der Formel 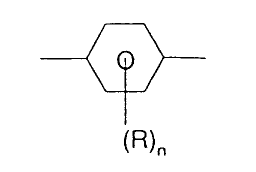
In dieser Formel steht R für einen Methyl-, Ethyl-, n-Propyl-, i.Propyl-, n-Butyl-, i-Butyl-, t-Butyl oder 1,1,3,3-Tetramethylbutylrest, Methoxy-, Ethoxy-, 2-Ethylhexyloxyrest oder Wasserstoff, vorzugsweise für Wasserstoff.

n bedeutet 1 bis 4, vorzugsweise 1 oder 2.

Vorzugsweise ist die Phenylengruppe unsubstituiert oder substituiert mit einer oder zwei Alkoxygruppen mit 1 bis 8 C-Atomen, insbesondere mit Methoxy, Ethoxy- oder 2-Ethylhexyloxygrupen.

Bevorzugte Verbindungen der Formel I sind diejenigen der Formeln I1 bis I11, wobei A¹ eine Gruppe der Formeln 1, 2 oder 3 und A² eine Gruppe der Formeln 1, 2 oder 3
ist und
- R: Alkyl oder Alkoxy mit 1 bis 10 C-Atomen bedeutet.

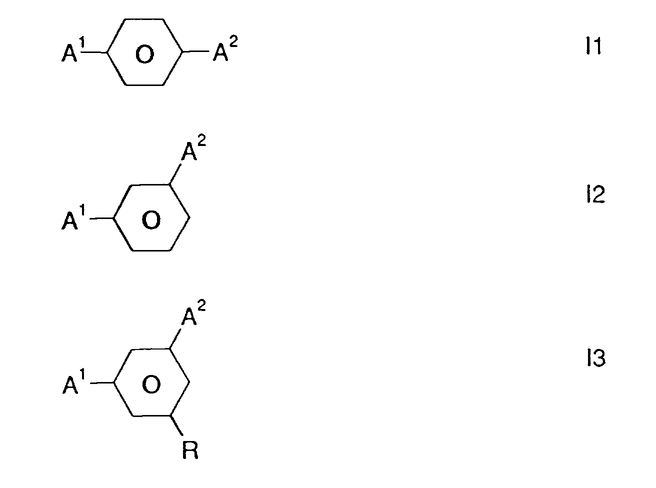

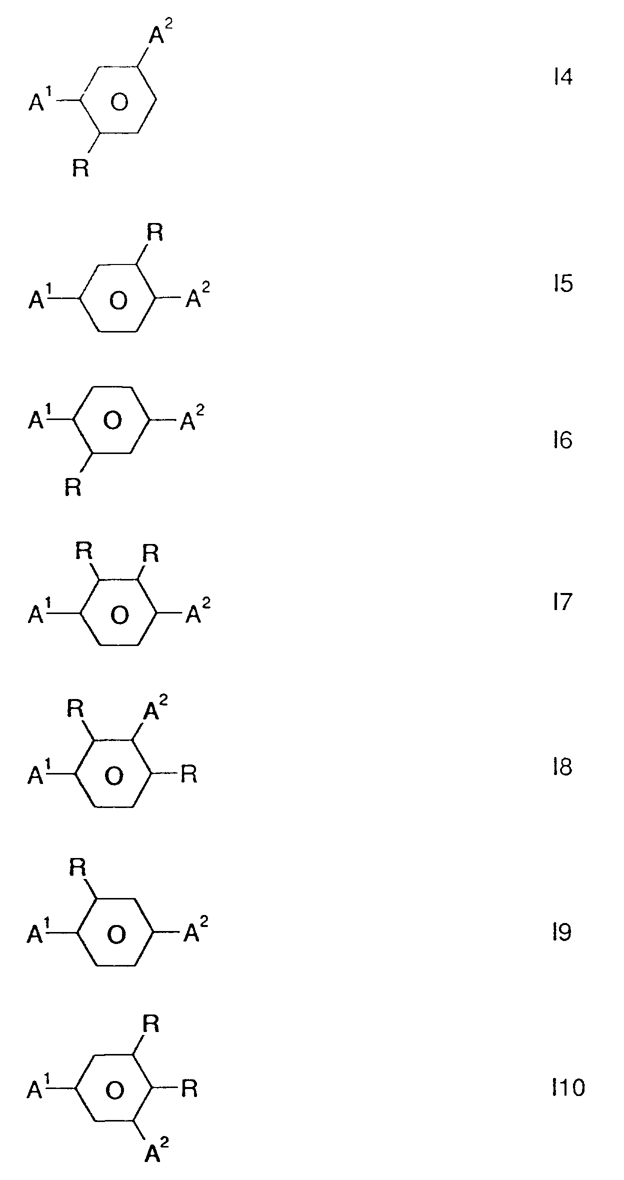

Darunter sind die Verbindungen der Formeln I1, I2, I3 und I4 besonders bevorzugt.

Weiterhin bevorzugt sind solche Verbindungen der Formel I sowie der Formeln I1 bis I10 worin A¹ Campher (Formel 1, R¹, R² = CH₃) und A² Norcampher (Formel 1, R¹, R² = H) bedeuten.

Man erhält die Verbindungen der Formel I z.B. dadurch, daß man ein Benzaldehyd-Derivat der Formel II,

YHC―Phe―CHO II

worin Phe die angegebene Bedeutung besitzt, und Y A¹ oder O bedeutet, in Gegenwart einer Base mit 8-Ketotricyclo[5.2.1.0^{2,6}]decan, Norcampher oder Chinuclidinon umsetzt.

Die Reaktion wird, in der Regel, in einem inerten Verdünnungsmittel, vorzugsweise einem protischen Lösungsmittel, insbesondere einem Alkohol, wie z.B. Methanol, Ethanol, Isopropanol oder tert. Butanol oder einem aprotischen Lösungsmittel, wie Diethylether, Toluol oder Cyclohexan oder Gemischen der genannten Lösungsmittel durchgeführt. Als Base setzt man vorzugsweise Alkalialkoholate, wie z.B. Natrium-Methylat, Natrium-Ethylat oder Kalium-tert.-Butylat ein.

Die Reaktion kann bei Temperaturen zwischen 0 °C und dem Siedepunkt des Reaktionsgemisches durchgeführt werden, vorzugsweise arbeitet man bei 25 bis 60 °C.

Die Aldehyde der Formel II sind bekannt oder werden nach bekannten Methoden hergestellt.

8-Ketotricyclo[5.2.1.0^{2,6}]decan, Norcampher und Chinuclidinon sind bekannt und käuflich erhältlich.

Man erhält die Verbindungen der Formel I, z.B. dadurch, daß man ein geschütztes Formyl-Benzaldehyd-Derivat der Formel III

(R³O)₂CH-Phe-CHO III

worin
- R³: Alkyl mit 1 bis 10 C-Atomen ist, oder
(R³O)₂CH eine 1,3-Dioxan-2-yl- oder eine 1,3-Dioxacyclopent-2-yl-gruppe
bedeutet,
in Gegenwart einer Base mit 8-Ketotricyclo[5.2.1.0^{2,6}]decan oder Chinuclidinon umsetzt, und das erhaltene Acetal der Formel IV

(R³O)₂CH-Phe-A² IV

in an sich bekannter Weise hydrolysiert.

Der erhaltene Methylidenbenzaldehyd der Formel II

OCH-Phe-A² II

wird in an sich bekannter Weise mit Campher, Norcampher, 8-Ketotricyclo[5.2.1.0^{2,6}]decan oder Chinuclidinon in Gegenwart einer Base umgesetzt.

Gegenstand der Erfindung ist auch das Verfahren zur Herstellung der neuen Verbindungen der Formel I.

Ein weiterer Gegenstand der Erfindung sind die neuen Zwischenprodukte der Formeln IV und V, worin A² eine Gruppe der Formel 2 oder 3 bedeutet.

Ein weiterer Gegenstand der Erfindung ist eine kosmetische Zubereitung, welche in einem kosmetisch verträglichen Träger eine wirksame Menge mindestens eines Derivates der obigen Formel I enthält.

Das erfindungsgemäße kosmetische Mittel kann als Mittel zum Schutz der menschlichen Epidermis oder der Haare oder als Sonnenschutzmittel verwendet werden.

Gegenstand der Erfindung ist ferner ein Verfahren zum Schutz der Haut und natürlich oder sensibilisierter Haare von Sonnenstrahlen, wobei auf die Haut oder die Haare eine wirksame Menge mindestens einer Verbindung der Formel I aufgetragen wird.

Mit "sensibilisierten Haaren" sind Haare gemeint, welche einer Dauerwellenbehandlung, einem Färbe- oder Entfärbeprozeß unterzogen worden sind.

Gegenstand der Erfindung ist ferner eine gefärbte oder ungefärbte lichtstabilisierte kosmetische Zubereitung, welche eine wirksame Menge mindestens eines Bis(methyliden)-phenylen-Derivates der obigen Formel I umfaßt.

Wird das erfindungsgemäße kosmetische Mittel als Mittel zum Schutz menschlicher Epidermis gegen UV-Strahlen verwendet, so liegt es in verschiedenen, für diesen Typ üblicherweise verwendeten Formen vor. So kann es insbesondere in Form öliger oder ölig-alkoholischer Lotionen, Emulsionen, wie als Creme oder als Milch in Form ölig-alkoholischer, ölig-wäßriger oder wäßrig-alkoholischer Gele oder als feste Stifte vorliegen oder als Aerosol konfektioniert sein.

Es kann kosmetische Adjuvanzien enthalten, welche in diese Art von Mittel üblicherweise verwendet werden, wie z.B. Verdickungsmittel, weichmachende Mittel, Befeuchtungsmittel, grenzflächenaktive Mittel, Konservierungsmittel, Mittel gegen Schaumbildung, Parfüms, Wachse, Lanolin, Treibmittel, Farbstoffe und/oder Pigmente, welche das Mittel selbst oder die Haut färben, und andere in der Kosmetik gewöhnlich verwendete Ingredienzien.

Die Verbindung der Formel I ist in der Regel in einer Menge von 0,5 bis 10 %, vorzugsweise 1 bis 8 %, insbesondere 1 bis 5 %, bezogen auf das Gesamtgewicht des kosmetischen Mittels zum Schutz menschlicher Epidermis, enthalten.

Man kann als Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglycol, Glycerin und Sorbit.

Eine bevorzugte Ausführungsform der Erfindung ist eine Emulsion, welche als Schutzcreme oder -milch vorliegt und außer der Verbindung der Formel I Fettalkohole, Fettsäureester, insbesondere Triglyceride von Fettsäuren, Fettsäuren, Lanolin, natürliche oder synthetische Öle oder Wachse und Emulgatoren in Anwesenheit von Wasser umfaßt.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glycols, wie Propylenglykol, und/oder eines Polyols, wie Glycerin, und Ölen, Wachsen und Fettsäureestern, wie Triglyceriden von Fettsäuren, dar.

Das erfindungsgemäße kosmetische Mittel kann auch als alkoholisches Gel vorliegen, welches einen oder mehrere Niedrigalkohole oder -polyole, wie Ethanol, Propylenglycol oder Glycerin, und ein Verdickungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäureestern, Lanolin und anderen Fettkörpern.

Gegenstand der Erfindung sind auch kosmetische Sonnenschutzmittel, die mindestens eine Verbindung der Formel I enthalten und andere UVB- und/oder UVA-Filter umfassen können.

In diesem Fall beträgt die Menge des Filters der Formel I in der Regel zwischen 1,0 und 8,0 Gew.% bezogen auf das Gesamtgewicht des Sonnenschutzmittels.

Ist ein Mittel als Aerosol konfektioniert, verwendet man in der Regel die üblichen Treibmittel, wie Alkane, Fluoralkane und Chlorfluoralkane.

Soll das erfindungsgemäße Mittel natürliche oder sensibilisierte Haare vor UV-Strahlen schützen, so kann es als Shampoo, Lotion, Gel oder Emulsion zum Ausspülen vorliegen, wobei die jeweilige Formulierung vor oder nach dem Shampoonieren, vor oder nach dem Färben oder Entfärben, vor oder nach der Dauerwelle aufgetragen wird; oder das Mittel liegt als Lotion oder Gel zum Frisieren und Behandeln, als Lotion oder Gel zum Bürsten oder Legen einer Wasserwelle, als Haarlack, Dauerwellmittel, Färbe- oder Entfärbemittel der Haare vor. Dieses Mittel kann außer der erfindungsgemäßen Verbindung verschiedene, in diesem Mitteltyp verwendete Adjuvantien enthalten, wie grenzflächenaktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien. Das Mittel enthält in der Regel 1,0 bis 5,0 Gew.% der Verbindung der Formel I.

Die vorliegende Erfindung befaßt sich auch mit kosmetischen Mitteln, die mindestens eine Verbindung der Formel I als Mittel zum Schutz vor UV-Strahlen und als Antioxidationsmittel enthalten; diese Mittel umfassen Haarproduke, wie Haarlacke, Wasserwell-Lotionen zum Einlegen der Haare, gegebenenfalls zum Behandeln oder leichteren Frisieren, Shampoos, Färbeshampoos, Haarfärbemittel, Schminkprodukte, wie Nagellack, Cremes und Öle zur Hautbehandlung, Make-up (fond de teint), Lippenstifte, Hautpflegemittel, wie Badeöle oder -cremes und andere kosmetische Mittel, die im Hinblick auf ihre Komponenten Probleme mit der Lichtstabilität und/oder der Oxidation im Laufe des Lagerns aufwerfen können.

Derartige Mittel enthalten in der Regel 1,0 bis 5,0 Gew.% einer Verbindung der Formel I.

Ferner befaßt sich die Erfindung mit einem Verfahren zum Schutz der kosmetischen Mittel vor UV-Strahlen und Oxidation, wobei diesen Mitteln eine wirksame Menge mindestens einer Verbindung der Formel I zugesetzt wird.

Ein weiterer Gegenstand der Erfindung ist die Verwendung der Verbindungen der Formel I als Sonnenfilter von großer Absorptionsbreite in einem Wellenlängenbereich von 320 bis 400 nm.

Ein weiterer Gegenstand der Erfindung ist die Verwendung der Verbindungen der Formel I als kosmetische Produkte.

Wie oben bereits erwähnt, hat die Anmelderin im Laufe ihrer Untersuchungen außerdem festgestellt, daß die Verbindungen der Formel I eine signifikante pharmakologische Aktivität auf dem Gebiet der Präventivbehandlung von Entzündungen und Hautallergien zeigen.

Gegenstand der Erfindung sind auch die Verbindungen der Formel I zur Verwendung als Medikament.

Gegenstand der Erfindung ist ferner ein pharmazeutisches Mittel, welches eine wirksame Menge mindestens einer Verbindung der Formel I als Wirkstoff in einem nicht-toxischen Träger oder Exzipienten enthält.

Das erfindungsgemäße pharmazeutische Mittel kann oral oder topisch verabreicht werden.

Für die orale Verabreichung liegt das pharmazeutische Mittel in Form von Pastillen, Gelatinekapseln, Dragees, als Sirup, Suspension, Lösung, Emulsion etc. vor. Zur topischen Verabreichung liegt es als Salbe, Creme, Pomade, Lösung, Lotion, Gel, Spray, Suspension etc. vor.

Dieses Mittel kann inerte oder pharmakodynamisch aktive Additive enthalten, insbesondere Hydratisierungsmittel, Antibiotika, steroide oder nicht-steroide antiinflammatorische Mittel, Carotinoide und Mittel gegen Psoriasis.

Dieses Mittel kann auch Geschmacksverbessetungsmittel, Konservierungsmittel, Stabilisatoren, Feuchtigkeitsregulatoren, pH-Regulatoren, Modifikatoren für den osmotischen Druck, Emulgatoren, Lokalanästhetika, Puffer etc. enthalten.

Es kann außerdem in an sich bekannter Weise in Retard-Form oder in einer Form konditioniert sein, in welcher der Wirkstoff rasch freigesetzt wird.

Auch ohne weitere Ausführungen wird davon ausgegangen, daß ein Fachmann die obige Beschreibung in weitestem Umfang nutzen kann. Die bevorzugten Ausführungsformen sind deswegen lediglich als beschreibende, keineswegs als in irgendeine Weise limitierte Offenbarung aufzufassen.

Die vollständige Offenbarung aller vor- und nachstehend aufgeführten Anmeldungen. Patente und Veröffentlichungen sind durch Bezugnahme in diese Anmeldung eingeführt.

Die nachfolgenden Beispiele sind repräsentativ für die vorliegende Erfindung.

### Beispiel 1

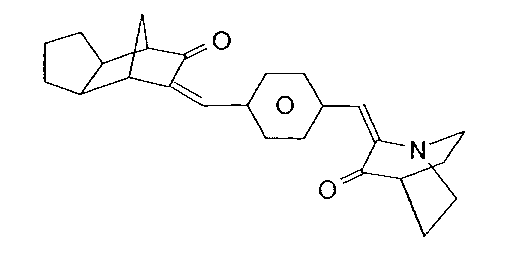

### 1A

### 9-(4-Formylbenzyliden)-8-ketotricyclo[5.2.1.0^{2,6}]decan

Eine Suspension aus 40 mMol (6,3 g) 8-Ketotricyclo[5.2.1.0^{2,6}]decan und 60 mMol Natrium-Methylat (11 g einer 30%igen Lösung) in 50 ml Cyclohexan werden 30 Minuten bei 50 °C gerührt, anschließend werden 50 mMol (9,0 g) Terephthalaldehydmonodimethylacetal eingetropft und 1 Stunde zum Rückfluß erhitzt. Danach wird auf Raumtemperatur abgekühlt und 100 ml Wasser hinzugegeben.

Die Phasen werden getrennt, die wäßrige Phase wird mit Cyclohexan extrahiert.

Nach Eindampfen unter reduziertem Druck wird der Rückstand mit Aceton aufgenommen, mit verdünnter Salzsäure versetzt und 1 Stunde zum Sieden erhitzt. Nach Abdestillation des Lösungsmittels erhält man 1A, welches ungereinigt weiterverarbeitet wird.

### 1B

### 1-(8-Ketotricyclo[5.2.1.0^{2,6}]dec-9-ylidenmethyl)-4-(chinuclidinon-6-ylidenmethyl)-benzol

Eine Suspension aus 40 mMol (5,0 g) Chinuclidinon wird analog Beispiel 2A mit 10 g 1A in Gegenwart von Natrium-Methylat umgesetzt. Nach üblicher Aufarbeitung und Umkristallisation erhält man 7,5 g = 50.4 % des reinen Produkts.

| Elementaranalyse: | | | | |
|---|---|---|---|---|
| berechnet: | C: 80,12 | H: 7,24 | O: 8,61 | N: 4,03 |
| gefunden: | C: 80,16 | H: 7,26 | O: 8,55 | N: 4,03 |

UV (Ethanol, c = 1 mg/100 ml): λₘₐₓ = 349 nm
Analog werden hergestellt: 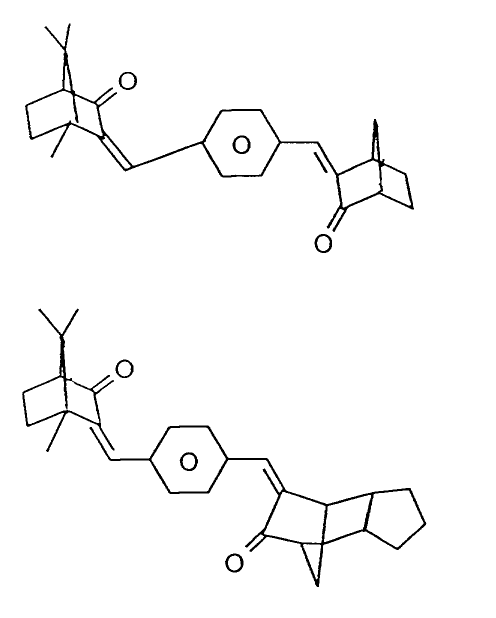
UV (Ethanol, c = 1 mg/100 ml): λₘₐₓ = 336 nm
E = 0.93

### Beispiel 2

### Sonnenschutzcreme (W/O)

| | | | % |
|---|---|---|---|
| A | Verbindung von Beispiel 1 | (1) | 3,00 |
| | Arlacel 581 | (2) | 7,00 |
| | Paraffinöl dünnflüssig (Art.-Nr. 7174) | (1) | 6,00 |
| | Arlamol S 7 | (2) | 2,00 |
| | Lunacera M | (3) | 5,00 |
| | Dow Corning 344 | (4) | 4,00 |
| | Miglyol 812 | (5) | 2,00 |
| | Oxynex 2004 (Art.-Nr. 6940) | (1) | 0,05 |
| B | Glycerin (Art.-Nr. 4093) | (1) | 2,00 |
| | Magnesium-Heptahydrat (Art.-Nr. 5882) | (1) | 0,17 |
| | Konservierungsmittel | | q.s. |
| | Wasser, demineralisiert | | ad 100,00 |

### Herstellung:

Phase A auf 75 °C, Phase B auf 80 °C erhitzen. Phase B langsam in Phase A einrühren. Homogenisieren. Unter Rühren abkühlen. Gegebenenfalls bei 40 °C parfümieren.

### Bezugsquellen:

(1) E. Merck, Darmstadt
(2) ICI, Essen
(3) LW Fuller, Lüneburg
(4) Dow Corning, Düsseldorf
(5) Hüls Troisdorf AG, Witten

### Beispiel 3

### Sonnenschutzcreme (O/W)

| | | | % |
|---|---|---|---|
| A | Verbindung von Beispiel 1 | (1) | 3,00 |
| | Emulgator E 2155 | (2) | 8,00 |
| | Stearinsäure (Art.-Nr.671) | (1) | 2,00 |
| | Paraffinöl flüssig (Art.-Nr. 7162) | (1) | 6,00 |
| | Paraffin schüttfähig (Art.-Nr. 7158) | (1) | 6,00 |
| | Cetylalkohol (Art.-Nr. 989) | (1) | 2,50 |
| | Miglyol 812 | (3) | 9,50 |
| | Abil AV 200 | (2) | 0,50 |
| | Cetylpalmitat (Art.-Nr. 15419) | (1) | 5,50 |
| | (Tocopherolacetat (Art.-Nr. 500952) | (1) | 0,05 |
| B | Glycerin (Art.-Nr. 4093) | (1) | 3,00 |
| | Propandiol-1,2 (Art.-Nr. 7478) | (1) | 2,00 |
| | Karion F flüssig (Art.-Nr. 2993) | (1) | 5,00 |
| | Allantoin (Art.-Nr. 1015) | (1) | 0,25 |
| | Triethanolamin (Art.-Nr. 8377) | (1) | 0,50 |
| | Konservierungsmittel | | q.s. |
| | Wasser, demineralisiert | | ad 100,00 |

### Herstellung:

Phase A auf 75 °C, Phase B auf 80 °C erhitzen. Phase B langsam in Phase A einrühren. Homogenisieren. Unter Rühren abkühlen. Gegebenenfalls bei 40 °C parfümieren.

### Bezugsquellen:

(1) E. Merck, Darmstadt
(2) Th. Goldschmidt, Essen
(3) Hüls Troisdorf AG, Witten

### Beispiel 4

### Sonnenschutzmilch (W/O)

| | | | % |
|---|---|---|---|
| A | Verbindung von Beispiel 1 | (1) | 3,00 |
| | Pionier L-15 | (2) | 19,00 |
| | Paraffinöl dickflüssig (Art.-Nr. 7160) | (1) | 15,00 |
| B | Glycerin (Art.-Nr. 4093) | (1) | 5,00 |
| | Magnesium-Heptahydrat (Art.-Nr. 5882) | (1) | 0,50 |
| | Konservierungsmittel | | q.s. |
| | Wasser, demineralisiert | | ad 100,00 |

### Herstellung:

Phase A auf 75 °C, Phase B auf 80 °C erhitzen. Phase B langsam in Phase A einrühren. Homogenisieren. Unter Rühren abkühlen. Gegebenenfalls bei 40 °C parfümieren.

### Bezugsquellen:

(1) E. Merck, Darmstadt
(2) Hansen & Rosenthal, Hamburg

### Beispiel 5

### Sonnenschutzmilch (O/W)

| | | | % |
|---|---|---|---|
| A | Verbindung von Beispiel 1 | (1) | 3,00 |
| | Eumulgin B 1 | (2) | 3,00 |
| | Cutina MD | (2) | 8,00 |
| | Miglyol 812 | (3) | 7,00 |
| B | Glycerin (Art.-Nr. 4093) | (1) | 5,00 |
| | Konservierungsmittel | | q.s. |
| | Wasser, demineralisiert | | ad 100,00 |

### Herstellung:

Phase A auf 75 °C, Phase B auf 80 °C erhitzen. Phase B langsam in Phase A einrühren. Homogenisieren. Unter Rühren abkühlen. Gegebenenfalls bei 40 °C parfümieren.

### Bezugsquellen:

(1) E. Merck, Darmstadt
(2) Henkel, Düsseldorf
(3) Hüls Troisdorf AG, Witten

### Beispiel 6

### Sonnenschutzöl

| | | | % |
|---|---|---|---|
| A | Verbindung von Beispiel 1 | (1) | 3,00 |
| | Arlatone T | (2) | 2,00 |
| | Miglyol 812 | (3) | 16,00 |
| | Cetiol B | (4) | 22,50 |
| | Isopropylmyristat | (4) | 7,50 |
| | Paraffinöl dünnflüssig (Art.-Nr. 7174) | (1) | 48,85 |
| | Oxynex 2004 (Art.-Nr. 6940) | (1) | 0,05 |
| B | Parfümöl | (5) | 0,10 |

### Herstellung:

Phase A unter Rühren auf 70 °C erhitzen bis alle Komponenten gelöst sind, kaltrühren und bei 40 °C Phase B zugeben.

### Bezugsquellen:

(1) E. Merck, Darmstadt
(2) ICI, Essen
(3) Hüls Troisdorf AG, Witten
(4) Henkel, Düsseldorf
(5) Haarmann & Reimer, Holzminden

## Patentansprüche

1. Bismethylidenphenylen-Derivate der Formel I,
A¹-Phe-A² I
worin
Phe eine unsubstituierte oder durch 1 bis 4 Hydroxy-, Alkyl- oder Alkoxygruppen mit 1 bis 10 C-Atomen substituierte Phenylengruppe,
A¹ eine Gruppe ausgewählt aus den Formeln 1, 2, 3 und 4 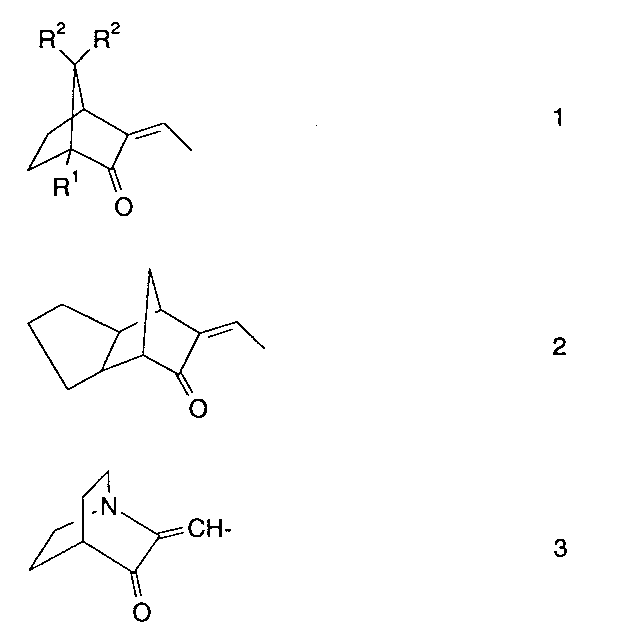 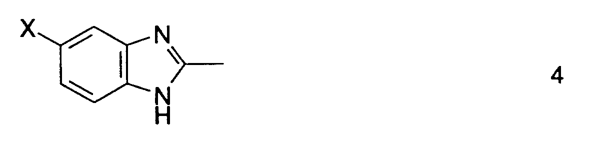
worin
R¹ H, CH₃ oder -CH₂SO₃H,
R² H oder CH₃, und X H oder SO₃H bedeuten,
und
A² eine von A¹ verschiedene Gruppe ausgewählt aus den Formeln 1, 2, 3 und 4
bedeuten.

2. Derivate nach Anspruch 1, worin Phe eine unsubstituierte oder durch 1 bis 2 Alkoxygruppen mit 1 bis 10 C-Atomen substituierte Phenylengruppe bedeutet.

3. Verfahren zur Herstellung von Verbindungen der Formel I, worin A¹ und A² jeweils eine Gruppe der Formel 2 oder 3 bedeuten, dadurch gekennzeichnet, daß man ein Formyl-Benzaldehyd-Derivat der Formel II,
YHC―Phe―CHO II
worin Phe die in Anspruch 1 angegebene Bedeutung hat, und Y A¹ oder O bedeutet, in Gegenwart einer Base mit 8-Ketotricyclo[5.2.1.0^{2,6}]decan oder Chinuclidinon umsetzt.

4. Kosmetische Zubereitung, dadurch gekennzeichnet, daß sie ene wirksame Menge mindestens einer Verbindung der Formel I nach Anspruch 1 in einem kosmetisch verträglchen Träger enthält.

5. Kosmetische Zubereitung nach Anspruch 4, dadurch gekennzeichnet, daß sie zusätzlich einen UV-B-Filter enthält.

6. Verwendung der Formel I nach einem der Ansprüche 1 bis 2 als kosmetisches Produkt.

7. Verbindung der Formel I nach einem der Ansprüche 1 bis 2 zur Verwendung als Arzneimittel.

8. Verbindung der Formel I zur Verwendung in der Prävention bestimmter Krebsarten.

9. Pharmazeutische Zubereitung, dadurch gekennzeichnet, daß sie eine wirksame Menge mindestens einer Verbindung der Formel I nach einem der Ansprüche 1 bis 2 in einem physiologisch unbedenklichen Träger oder Exzipienten enthält.

10. Verwendung der Verbindungen der Formel I nach einem der Ansprüche 1 bis 2 als Arzneimittel.
